# EUROPEAN PATENT SPECIFICATION

(11) **EP 2 533 751 B1**
(45) Date of publication and mention of the grant of the patent: **15.05.2019**
(21) Application number: 11742604.9
(22) Date of filing: 25.01.2011
(51) Int. Cl.: A61K 8/02, A61Q 11/00, A61K 8/96

(54) **COMPOSITION FOR CLEANING TEETH COMPRISING NATURAL GLASS AND RELATED METHODS**
ZUSAMMENSETZUNG ZUR ZAHNREINIGUNG MIT NATÜRLICHEM GLAS UND ENTSPRECHENDE VERFAHREN
COMPOSITION POUR LE NETTOYAGE DE DENTS COMPRENANT DU VERRE NATUREL ET PROCÉDÉS S'Y RAPPORTANT

(30) Priority: 11.02.2010 US 303487 P
(43) Date of publication of application: 19.12.2012
(73) Proprietor: Imerys Filtration Minerals, Inc., San Jose, CA 95112 (US)
(72) Inventor: WANG, Bo, Lompoc CA 93436 (US)
(74) Representative: Rushton, David John
(86) International application number: PCT/US2011/022317
(87) International publication number: WO 2011/100104

(56) References cited:
- WO-A1-00/74638
- WO-A2-01/45646
- US-A- 5 597 553
- US-A- 5 597 553
- US-A- 5 891 473
- US-A1- 2003 224 702
- US-A1- 2006 075 930
- US-A1- 2008 160 055
- US-A1- 2010 136 069
- US-B1- 6 280 707

## Description

### CLAIM OF PRIORITY

This PCT International Application claims the benefit of priority of U.S. Provisional Patent Application No. 61/303,487, filed February 11, 2010.

### FIELD OF THE DISCLOSURE

The present disclosure relates to compositions for cleaning teeth including natural glass and related methods. In particular, the present disclosure relates to compositions for cleaning teeth including natural glass having the particle size characteristic as defined in the present claims.

### BACKGROUND OF THE DISCLOSURE

Dental care includes the use of compositions for cleaning teeth, such as, for example, dentifrice compositions such as pastes or powders for cleaning teeth. Toothpaste is a commonly known example of a dentifrice composition, which typically has a paste-like form and which may include one or more components, such as, for example, binders, humectants, abrasives, detergents, flavoring agents, and preventatives, such as anti-infective agents and/or other medicaments.

The abrasive component in toothpaste serves to improve its cleaning effectiveness. However, while abrasives may improve cleaning effectiveness, they may also lead to undesirable erosion of the teeth.

One composition that has been added to toothpaste to improve its cleaning effectiveness is perlite. Perlite is an example of a naturally-occurring glass, such as, for example, an amorphous volcanic glass having a relatively high water content. By virtue of its relatively high water content, perlite expands when heated, for example above about 850-900°C. While perlite may improve the cleaning effectiveness of toothpaste, it may also lead to premature degradation of teeth due to its inherently abrasive nature. US 5597553 A relates to toothpaste comprising expanded perlite generally with a particle size in the range of 1 to 150 *µ*m.

Thus, it may be desirable to provide compositions that assist with the effectiveness of cleaning teeth, but which do not lead to premature erosion of the teeth due to excessive abrasiveness.

### SUMMARY

In the following description, certain aspects and embodiments will become evident. It should be understood that the aspects and embodiments, in their broadest sense, could be practiced without having one or more features of these aspects and embodiments. Thus, it should be understood that these aspects and embodiments are merely exemplary.

The present invention is defined in and by the appended claims. One aspect of the disclosure relates to a composition for cleaning teeth. The composition includes natural glass, wherein the natural glass has a top particle size (d₉₀) ranging from 20 *µ*m to less than 50 micrometers (*µ*m) and a median particle size (dso) less than 30 *µ*m and wherein the natural glass ranges from 0.1 percent to 25 percent by weight of the composition and comprises perlite. Top particle size (d₉₀) is defined as the size for which 90 percent of the volume of the particles is smaller than the indicated size. Median particle size (d₅₀) is defined as the size for which 50 percent of the volume of the particles is smaller than the indicated size.

The natural glass may exhibit a relative dentin abrasion (RDA) value less than 200. RDA testing is a method of measuring of the erosive effect on tooth dentin of abrasives in compositions for cleaning teeth, and RDA value is standardized in accordance with DIN/ISO standard 11609, a standard that has been adopted by the American Dental Association (ADA). Higher RDA values indicate higher levels of abrasiveness.

The natural glass comprises perlite. For example, the natural glass may include expanded perlite, for example, milled, expanded perlite. The perlite may include unexpanded perlite.

The natural glass may have a top particle size (d₉₀) less than 40 *µ*m, for example, a top particle size (d₉₀) less than 35 *µ*m. The natural glass may have a top particle size (d₉₀) ranging from 30 *µ*m to 40 *µ*m.

The natural glass may have a median particle size (d₅₀) less than 25 *µ*m, for example, a median particle size (d₅₀) ranging from 15 *µ*m to 25 *µ*m.

The natural glass ranges from 0.1 percent to 25 percent by weight of the composition, or, for example, from 0.1 percent to 10 percent by weight of the composition.

The natural glass can have a blue light brightness of greater than 70. The natural glass can have a whiteness (L-value) of greater than 75, such as, for example, greater than 80. The natural glass can have a yellowness (b-value) of less than 5, such as, for example, less than 2. The natural glass can have a redness (a-value) of less than 1.0, such as, for example, less than 0.5.

The composition may exhibit an RDA value less than 230, for example, an RDA value less than 200. The composition may exhibit a pellicle cleaning ratio (PCR) value of at least 110, for example, a PCR value of at least 120. The PCR test is a laboratory method accepted by the ADA as useful in characterizing the stain cleaning actions of compositions for cleaning teeth. Test data are referenced against that of the ADA's reference material, calcium pyrophosphate, with the stain reduction resulting from calcium pyrophosphate being taken to be by definition 100. Higher values of PCR indicate greater stain removal or "whitening." The composition may exhibit tooth polishing effect.

The composition may include a toothpaste base. For example, the toothpaste base may include at least one of binders (thickeners), humectants, abrasives, detergents, flavoring agents, and preventatives. The natural glass may exhibit toothpaste thickening effect.

Additional objects and advantages of the disclosure will be set forth in part in the description which follows, and in part will be obvious from the description, or may be learned by practice of the disclosed embodiments.

Aside from the arrangements set forth above, the embodiments could include a number of other arrangements, such as those explained hereinafter. It is to be understood that both the foregoing description and the following description are exemplary only.

### BRIEF DESCRIPTION OF THE DRAWINGS

The accompanying drawings, which are incorporated in and constitute a part of this description, illustrate several exemplary embodiments and together with the description, serve to explain the principles of the embodiments. In the drawings,
Fig. 1 is a scanning electron micrograph of coarse fraction of a classified, expanded perlite.
Fig. 2 is a scanning electron micrograph of fine fraction of a classified, expanded perlite sample according to an exemplary embodiment;
Fig. 3 is a graph showing Relative Dentin Abrasion (RDA) test results for three examples of natural glass vs. top particle size (d₉₀); and
Fig. 4 is a graph showing Pellicle Cleaning Ratio (PCR) test results for three examples of natural glass vs. top particle size (d₉₀).

### DESCRIPTION OF EXEMPLARY EMBODIMENTS

Reference will now be made in more detail to a number of exemplary embodiments of the invention.

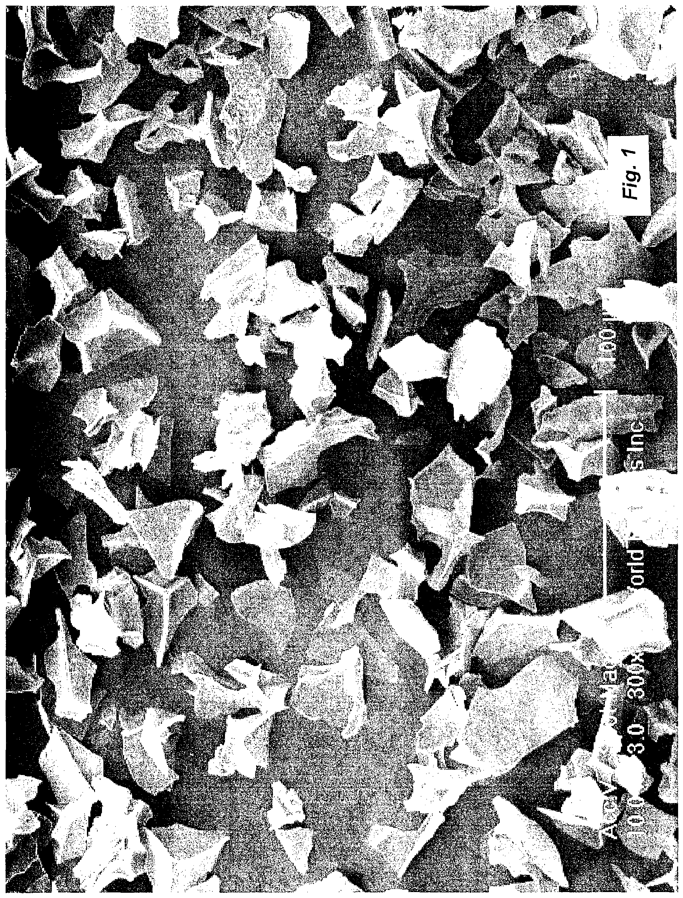

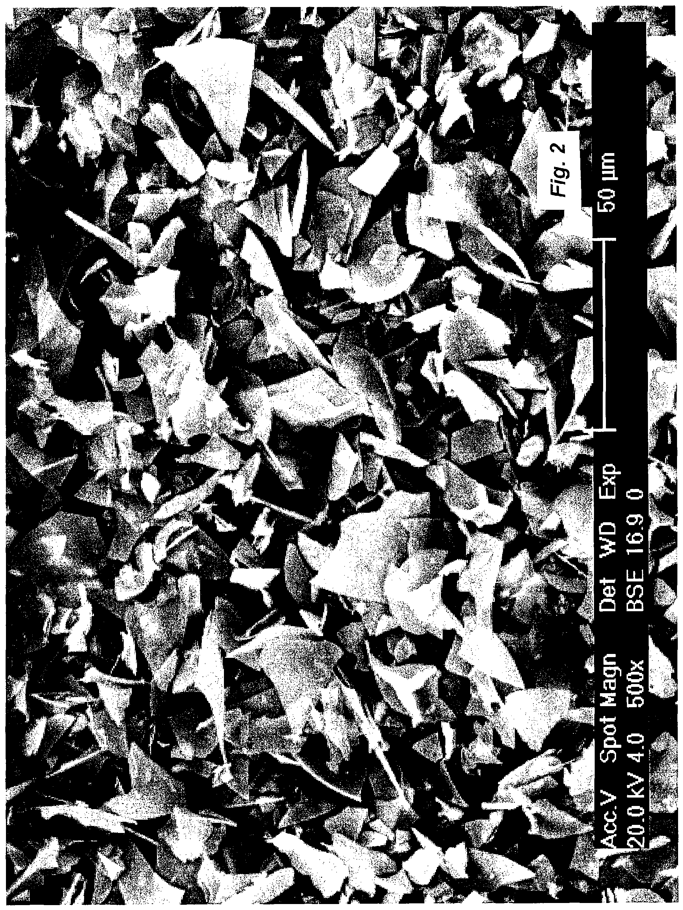

Fig. 1 shows an example of large particles of natural glass from a coarse fraction of a classified product, in particular, expanded perlite. As can be seen in Fig. 1, perlite particles having a size greater than about 50 *µ*m tend to be generally three-dimensional, multi-angular particles. In contrast, as shown in Fig. 2 fine perlite particles from the fine fraction of the classified product having a size less than about 50 *µ*m tend to be generally two-dimensional and relatively more platy than the larger particles. Thus, it is believed that natural glass particles (e.g., expanded perlite particles) having a size greater than about 50 *µ*m tend to be more abrasive than particles having a smaller size. Further, it is also believed that smaller, platy particles tend to break down to even smaller particles more easily during, for example, a cleaning process.

According to some exemplary embodiments, natural glass, for example, commercially-available natural glass such as expanded perlite, may be milled and classified, such that the milled and classified natural glass has a top particle size (d₉₀) less than 50 *µ*m. Dynamic classifiers (e.g., mechanical air classifiers), static classifiers (e.g., cyclones), and sieving can be used to control the top particle size of the expanded perlite. For example, an expanded perlite having a top particle size (d₉₀) of 112 *µ*m, a median particle size (d₅₀) of 60 *µ*m, and a (d₁₀) particle size of 22 *µ*m may be milled and/or classified according to methods known to those skilled in art to obtain perlite having a top particle size (d₉₀) less than 50 *µ*m. (A particle size designated "(d₁₀)" is defined as the size for which 10 percent of the volume of the particles is smaller than the indicated size.) For example, the milled and/or classified natural glass may have a top particle size (d₉₀) less than 45 *µ*m, such as, for example, a top particle size (d₉₀) less than 40 *µ*m or less than 30 *µ*m. According to some embodiments, the natural glass has a top particle size (d₉₀) ranging from 20 *µ*m to 40 *µ*m, such as, for example, from 25 *µ*m to 35 *µ*m.

According to some exemplary embodiments, the milled and/or classified natural glass (e.g., expanded perlite) may have a median particle size (d₅₀) less than 30 *µ*m. For example, the natural glass may have a median particle size (d₅₀) less than 25 *µ*m, such as, for example, a median particle size (d₅₀) less than 20 *µ*m. Some embodiments have a median particle size (d₅₀) ranging from 5 *µ*m to 25 *µ*m, such as, for example, from 10 *µ*m to 20 *µ*m.

Fig. 2 shows an example of an expanded perlite that has been milled and/or classified in the exemplary manner described above, and it shows the relatively two-dimensional and platy nature of the milled and/or classified perlite relative to the coarse perlite shown in Fig. 1. As explained in more detail herein, compositions for cleaning teeth that include natural glass with these exemplary particle size characteristics may result in compositions that result in effective cleaning of the teeth without adversely increasing the abrasiveness of the composition. It is believed that this may result from the relatively smaller natural glass particles having a relatively platy characteristic that increases the area of contact with the tooth relative to the sharp point-like contact of the three-dimensional and angular nature of relatively larger natural glass particles.

Some embodiments of compositions for cleaning teeth include natural glass in an amount ranging from, for example, 0.1 percent to 25 percent by weight of the weight of the composition, for example, from 0.1 percent to 15 percent by weight. According to some embodiments, natural glass may be present in an amount ranging from, for example, 0.1 percent to 10 percent by weight of the composition, or, for example, from 0.1 percent to 3 percent by weight.

According to some embodiments, a composition for cleaning teeth includes natural glass, wherein the natural glass is perlite. For example, according to some embodiments, the natural glass is expanded perlite. The perlite may be milled and classified, expanded perlite. According to some embodiments, the natural glass is unexpanded perlite.

Exemplary embodiments of compositions for cleaning teeth include natural glass and exhibit an RDA value less than 220. For example, some embodiments of compositions for cleaning teeth include natural glass and exhibit an RDA value less than 200, for example, less than 180. Some exemplary embodiments of compositions for cleaning teeth include natural glass and exhibit a PCR value of at least 110. For example, some embodiments include natural glass and exhibit a PCR value of at least 120.

Some embodiments of compositions for cleaning teeth are dentifrice compositions. According to some embodiments, the dentifrice composition is toothpaste, in particular, a dentifrice including a toothpaste base. For example, the toothpaste base may include at least one ingredient chosen from binders, such as thickening agents and/or gelling agents, humectants, foaming agents such as detergents, and polishing agents. The toothpaste base may also contain at least one additional ingredient chosen from, for example, water, preservative agents, flavoring agents, sweeteners, and fluoride containing compounds. It will be readily apparent to the skilled artisan that the components and their relative amounts in the toothpaste base may be modified to achieve the desired toothpaste product.

The toothpaste base according to some embodiments may contain at least one binder, such as thickeners, which may also be referred to as gelling agents. Any art-recognized gelling or thickening agent may be used. Thickening or gelling agents may be selected from natural, synthetic, and gum-like materials, including, but not limited to, carboxyl methyl cellulose, carrageenin, xantham gum, bentonite, and hydrated silica. The at least one thickening or gelling agent may be present in the toothpaste base in an amount ranging from, for example, about 0.1 percent to about 5 percent by weight, for example, from about 0.1 percent to about 3 percent by weight. According to some embodiments, the at least one thickening or gelling agent is present in the toothpaste base in an amount ranging from, for example, about 0.5 percent to about 1.5 percent by weight. Natural glass, such as classified expanded perlite having a smaller top particle size, can also be used as thickener.

According to some embodiments, the toothpaste base may also contain at least one ingredient chosen from detergents and surfactants. Suitable non-limiting examples of appropriate detergents for use in the toothpaste base include anionic surfactants, such as sodium alkylsulfates, sodium laurylsulfate, sodium myristylsulfate and sulfosuccinic acid surfactants; dialkyl sodium sulfosuccinate; non-anionic surfactants; and amphoteric surfactants. The at least one ingredient chosen from detergents and surfactants may be present in the toothpaste base in an amount ranging from, for example, about 0.1 percent to about 10 percent by weight, for example, from about 0.1 percent to about 5 percent by weight, and further, for example, from about 0.5 percent to about 3 percent by weight.

According to some embodiments, the toothpaste base may also contain at least one humectant, such as, for example, humectants chosen from glycerin, sorbitol, propylene glycols, polyethylene glycols, and mixtures thereof. The at least one humectant may be present in the toothpaste base in an amount ranging from, for example, about 10 percent to about 90 percent by weight, for example, from about 20 percent to about 80 percent by weight. According to some embodiments, the at least one humectant may be present in an amount ranging from about 30 percent to about 70 percent by weight.

Some embodiments of toothpaste base may contain at least one coloring or whitening agent. Any art-recognized coloring or whitening agent may be used. Coloring and whitening agents may include, for example, titanium dioxide. Coloring or whitening agents may be present in the toothpaste base in an amount ranging from about 0.1 percent to about 5 percent by weight, for example, ranging from about 0.1 percent to about 3 percent by weight, or, for example, ranging from about 0.1 percent to about 1 percent by weight.

The toothpaste base according to some embodiments may contain at least one preservative. Any art-recognized preservative may be used. For example, preservatives may be selected from sodium benzoate and methyl paraben. Preservatives may be present in the toothpaste base in an amount ranging from, for example, about 0.1 percent to about 3 percent, by weight, for example, ranging from about 0.1 percent to about 1 percent by weight, and further, for example, from about 0.1 percent to about 0.5 percent by weight. The toothpaste composition may further contain at least one additional ingredient chosen from therapeutic ingredients and preventatives such as water-insoluble non-cationic antibacterial agents, for example, triclosan, and cationic antibacterial agents.

The toothpaste base may also contain at least one foaming agent. Any art-recognized foaming agent may be used, and appropriate foaming agents will be readily apparent to the skilled artisan. Further, the toothpaste base may contain at least one flavoring agent. Any art-recognized flavoring agent may be used, and appropriate flavoring agents will be readily apparent to the skilled artisan. For example, flavoring agents may be chosen from oils of spearmint, peppermint, wintergreen, sassafras, clove, sage, eucalyptus, cinnamon, lemon, orange, and methyl salicylate.

The toothpaste base may contain at least one sweetener. Any art-recognized sweetener may be used, and appropriate sweeteners will be readily apparent to the skilled artisan. For example, sweeteners may be chosen from at least one of sucrose, lactose, maltose, xylitol, sodium cyclamate, perillartine, aspartyl phenyl alanine methyl ester, and saccharine.

The toothpaste base may contain fluoride, such as, any compatible composition that will dissociate and release fluorine-containing ions in water. Fluoride compositions may be chosen from one or more of sodium fluoride, stannous fluoride, sodium monofluorophosphate, potassium fluoride, potassium stannous fluoride, sodium fluorostannate, stannous chlorofluoride, and amine fluoride. Fluorides may be present in the toothpaste base in an amount ranging from about, for example, 0.1 percent to about 3 percent, by weight, for example, from about 0.1 percent to about 1 percent by weight, and further, for example, from about 0.2 percent to about 0.8 percent by weight.

Compositions according to some embodiments may also include abrasive materials chosen from any fluoride compatible abrasive material. Suitable non-limiting examples of abrasive materials that may be used may be chosen from, for example, natural glass, silica, alumina, aluminosilicate, dicalcium phosphate, sodium bicarbonate, sodium metaphosphate, potassium metaphosphate, tricalcium phosphate, calcium pyrophosphate, calcium carbonate, and bentonite. According to some embodiments, abrasives may be present in an amount ranging from about 4 percent to about 25 percent by weight, relative to the total weight of the composition.

### EXAMPLES

### Preparation of Examples

Three examples of natural glass were prepared and tested according to the following description. Examples 1-3 were prepared using a pilot scale Alpine™ 200 ATP air classifier (marketed by Hosokawa Alpine Aktiengesellchaft of Augsburg, Germany). Other dynamic classifiers (e.g., mechanical air classifiers) and other classification methods such as static classifiers (e.g., cyclones), and sieving (such as vibration screener and centrifugal sifter) known to those skilled in the art may be used. The mechanical air classifier used generally includes a horizontally mounted high speed classifying wheel and a classifying air outlet. Classifying air injected into the machine base flows inwards through the classifying wheel and discharges the fine particles, and coarse particles rejected by the classifying wheel are ejected from the classifier through a coarse material outlet. By adjusting operating parameters of the classifier, such as, for example, classifier wheel speed and air flow pressure, a material having the desired characteristics may be achieved.

Examples 1-3 were obtained from a commercially-available milled, expanded perlite product, Harborlite® 2000, which was used as the feed material for the exemplary classifier described above. The feed material had a median particle size (d₅₀) of 60 *µ*m, and a particle size distribution (PSD) from 22 *µ*m (d₁₀) to 112 *µ*m (d₉₀).

The particle size distribution of samples was determined in accordance with the phenomenon of scattered light from a laser beam projected through a stream of particles. The amount and direction of light scattered by the particles is measured by an optical detector array and then analyzed by a microcomputer, which calculates the size distribution of the particles in the sample stream. For example, the particle size data may be obtained on a Leeds and Northrup Microtrac X100 laser particle size analyzer (marketed by Leeds and Northrup of North Wales, Pennsylvania). This instrument is capable of determining particle size distribution over a particle size range from 0.12 *µ*m to 704 *µ*m.

The color of the natural glass was determined using Hunter scale "L," "a," and/or "b" color data collected on a Spectro/plus Spectrophotometer (Color and Appearance Technology, Inc., Princeton, New Jersey). The L-value indicates the level of lightness or darkness, the a-value indicates the level of redness or greenness, and the b-value indicates the level of yellowness or blueness. Blue light brightness was calculated from the L-, a-, and b-value data. A krypton-filled incandescent lamp was used as the light source. The instrument was be calibrated according to the manufacturer's instructions using a highly polished black glass standard and a factory-calibrated white opal glass standard.

Operating parameters such as classifier rotor speed and air flow pressure were adjusted to the values shown in Table 1 below to achieve Examples 1-3.

**Table 1**

| **Example** | **Rotor Speed (rpm)** | **Feed rate (kg/hr)** | **Primary Air (m³/h)** | **Secondary Air (m³/h)** | **Yield (%)** |
|---|---|---|---|---|---|
| Example 1 | 5100 | 210 | 410 | 490 | 52 |
| Example 2 | 4700 | 201 | 420 | 500 | 60 |
| Example 3 | 3700 | 211 | 430 | 500 | 72 |

The particle size distribution and color characteristics of Examples 1-3 are shown Table 2 below.

**Table 2**

| **Example** | **d₁₀** | **d₅₀** | **d₉₀** | **L** | **a** | **b** | **Blue Light Brightness** |
|---|---|---|---|---|---|---|---|
| Example 1 | 7.53 | 18.21 | 34.36 | 91.76 | -0.11 | 1.58 | 82.13 |
| Example 2 | 8.46 | 20.47 | 38.70 | 91.47 | -0.07 | 1.61 | 81.56 |
| Example 3 | 12.71 | 29.69 | 57.22 | 91.26 | -0.11 | 1.86 | 80.85 |

### Testing of Examples

### 1. Relative Dentin Abrasion Test on Dentifrices

The RDA value indicates the relative abrasion level of dentifrices. The RDA testing procedure used was the American Dental Association (ADA)-recommended procedure for determining dentifrice abrasivity. Dentin specimens were placed in a neutron flux under the controlled conditions outlined by the ADA. The specimens were then mounted in methylmethacrylate so they would fit in a V-8 cross-brushing machine. The specimens were brushed for a 1,500 stroke, precondition run using a slurry consisting of 10 grams of ADA reference material in 50 milliliters of a 0.5% carboxymethylcellulose (CMC) glycerine solution. The brushes used were those specified by the ADA, and brush tension was 150 grams.

Following a precondition run, the RDA test was performed using the above parameters (150 grams and 1,500 strokes) in a sandwich design in which each test material slurry (25 grams/40 milliliters of water) was flanked by the reference material slurries (10 grams/50 milliliters 0.5% CMC).

Samples of 1 milliliter were taken, weighed (0.01 grams) and added to 5 milliliters of scintillation cocktail. The samples were mixed well and immediately placed on the scintillation counter for radiation detection. Following counting, the net counter per minute (CPM) values were divided by the weight of the sample to calculate a net CPM/gram of slurry. The net CPM/gram of the pre- and post-ADA reference material for each test slurry was then calculated and averaged to use in the calculation for RDA for the test material. The ADA material was assigned a value of 100, and its ratio to the test material was calculated. The results of the RDA test are reported in Table 3 below. It is noteworthy to mention these RDA tests were not based on an actual toothpaste formulation, and the perlite loading percentage is significantly higher than would be used in a conventional toothpaste formulation. These test results provide relative RDA comparison between different natural glass (perlite in this case) products with different top particle sizes.

### 2. Stained Pellicle Removal - Pellicle Cleaning Ratio Test

The PCR value is an indication of the ability of dentifrices to remove stained pellicle (i.e., an indication of the cleaning ability of dentifrice formulations). Previous studies (J. Dent. Res., 61:1236, 1982) have indicated that the results of this test with dentifrice slurries compare favorably with those obtained in controlled clinical trials. Thus, the results of this test using dentifrice slurries may be considered to predict clinical findings with a reasonable degree of confidence.

During the PCR test, bovine, permanent, central incisors were cut to obtain enamel specimens measuring approximately 10 millimeters square. The enamel specimens were then embedded in an autopolymerizing methacrylate resin, so that only the enamel surfaces were exposed. The enamel surfaces were then smoothed and polished on a lapidary wheel and lightly etched to expedite stain accumulation and adherence. The specimens were then placed on a rotating rod in a 37°C incubator, alternately exposing them to air and to a staining broth solution consisting of trypticase soy broth, tea, coffee, mucin, FeCl3, and *Sarcina lutea* (bacteria). The staining broth was changed, and the specimens were rinsed daily for approximately seven days. After seven days, a darkly-stained pellicle film was apparent on the enamel surfaces. The specimens were then rinsed, allowed to air dry, and refrigerated until use.

For purposes of the PCR test, all dentifrice examples were tested using specimens prepared at the same time. The amount of in vitro stain was graded photometrically (i.e., via a Minolta C221, colorimeter) using only the L value of the LAB scale. The area of the specimens scored was a ¼ inch diameter circle in the center of the enamel specimen. Specimens with scores between 25 and 42 (with 25 being more darkly stained) were used. On the basis of these scores, the specimens were divided into groups of 16 specimens each, with each group having the same average baseline score.

The specimens were then mounted on a mechanical V-8 cross-brushing machine equipped with soft nylon-filament (Oral-B™ 40) toothbrushes. Tension on the enamel surface was adjusted to 150 grams. The dentifrice samples were used as slurries prepared by mixing 25 grams of dentifrice with 40 milliliters of deionized water. The ADA abrasion reference material (Ca₂P₂O₇) was prepared by mixing 10 grams of the reference material in 50 milliliters of a 0.5% CMC solution. The specimens were thereafter brushed for 800 strokes (i.e., for 4½ minutes). To minimize mechanical variables, one specimen per group was brushed on each of the eight brushing heads. Fresh slurries were made after being used to brush four specimens. Following brushing, specimens were rinsed, blotted dry, and scored again for stain, as previously described.

The difference between the pre- and post-brushing stain scores was determined, and the mean and standard error were calculated for the reference group. The cleaning ratio for the reference material group was assigned a value of 100. The mean decrement of the reference group was divided into 100 to obtain a constant value to multiply by each individual test decrement within the study. The individual cleaning ratio of each specimen was thereafter calculated by multiplying the decrement by the constant. The results of the PCR test are summarized in the Table 3 below.

**Table 3**

| **Sample ID** | **d₉₀** | **RDA (Relative Dentin Abrasion)** | **PCR (Pellicle Cleaning Ratio)** |
|---|---|---|---|
| Example 1 | 34.36 | 172.12 ± 2.93 | 121.19 ± 6.76 |
| Example 2 | 38.70 | 200.54 ± 2.91 | 126.66 ± 7.47 |
| Example 3 | 57.22 | 261.55 ± 9.02 | 129.40 ± 8.29 |

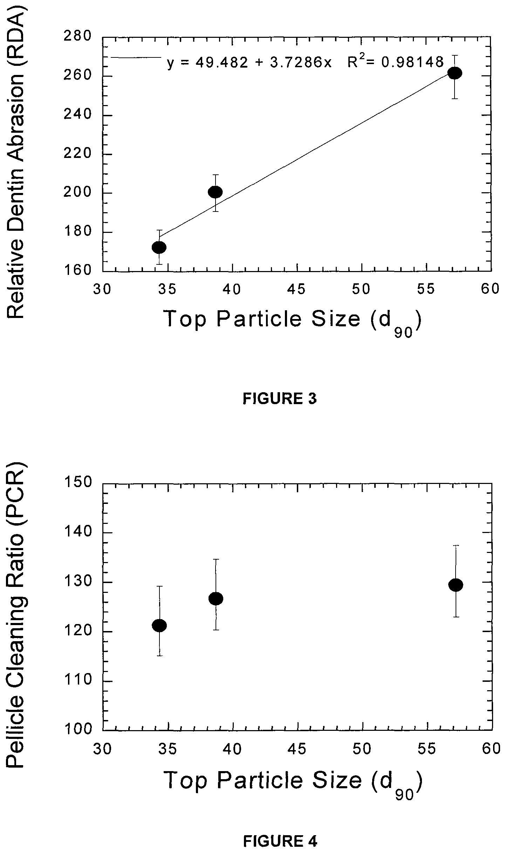

Table 3 and Fig. 3 show that abrasion decreases significantly with decreasing top particle size (d₉₀). For example, about a 30% reduction in abrasion can be achieved when top particle size (d₉₀) is less than 40 *µ*m. As shown in Fig. 1, the perlite particles larger than 50 *µ*m are generally three-dimensional and multi-angular in nature. Such particles tend to be more abrasive as compared to perlite particles that are less than 50 *µ*m, which are generally two-dimensional and platy in nature. It is believed that platy particles tend easily break down during a cleaning process, which reduces abrasion. Table 3 also shows that the identified commercial baghouse perlite products have a top particle size (d₉₀) ranging from 57 *µ*m to above 72 *µ*m. It is believed that when particles 50 *µ*m and larger are removed, abrasion can be significantly reduced.

Table 3 and Fig. 3 show that even with the smaller top particle size, Examples 1-3 are still effective in cleaning teeth. It is believed that this may be due to a greater contact area obtained with the relatively more platy surface of the smaller particles relative to the point-type contact with larger particles, which are relatively more three-dimensional and angular in character. Thus, compositions for cleaning teeth including natural glass having a smaller top particle size provide effective cleaning and reduced erosion of the teeth.

### 3. Polishing Effectiveness Test

The effectiveness of a milled and classified expanded perlite with smaller particle top size was measured using a polishing effectiveness test similar to that described in a paper by Bailey and Phillips (J. Dent. Res, 29:740, 1950) on the study of abrasive prophylactic agents and techniques on enamel surfaces.

The toothpaste samples for the polishing test contained sorbitol, water, perlite, hydrated silica (HCS - high cleaning silica), hydrated Silica (thickener), PEG, titanium dioxide, sodium lauryl sulfate, flavor, sodium saccharin, tetrasodium pyrophosphate, sodium fluoride, cellulose gum. Toothpaste sample 1 contained 20% high cleaning silica and Toothpaste sample 2 contained 2% perlite and 18% high cleaning silica. The perlite used in toothpaste sample 2 was a classified expanded perlite prepared by a method comparable to that of Example 1 above, and having a top particle size (d₉₀) of 35 microns.

Bovine permanent, central incisors were cut to obtain labial enamel specimens approximately 10 x 10 mm². The enamel specimens were embedded in an autopolymerizing methacrylate resin so that only the enamel surfaces were exposed. The enamel surfaces were smoothed and polished on a lapidary wheel. The bovine samples were prescreened by prophying them with a water slurry of LPA-3T abrasive (e.g., an aluminum oxide optical finishing powder) to a high luster.

The bovine samples were scored for surface reflectance using a beam reflectometer. Specimens were placed under the light source and the entire enamel labial surface was scanned observing the highest reading (the highest polished area). A polish score of at least 7.0 was achieved before any specimen is accepted for use in the study. This is based on a black onyx reference block being set on a reflectance value of 6.0. This procedure was used to confirm the specimens' ability to achieve a high polish.

The specimens were etched by decalcifying them in 1% HCL (v/v) for 2 minutes to provide a dull surface to initiate the study. The subsequent reflectometer baseline reading was around 2.0. The specimens were also scored using the Novo-Curve Glossmeter. The specimens were scored with the glossmeter after the reflectometer reading had been performed and then rotated 180 and scored again. The average of the two scores was used to calculate the glossmeter data.

Following the baseline (etched) scoring; the specimens were placed on a cross-brushing machine. The brush tension was adjusted to 150 grams, and the specimens were brushed for 4,500 strokes with the appropriate dentifrice slurry (25 grams of dentifrice + 40 grams of deionized water, 10 grams of powder + 50 ml 0.5% CMC) and a medium brush (Oral B-40). The specimens were removed from the brushing machine, rinsed and scored with both the reflectometer and the glossmeter once again for polish. Etching dulled the specimens again and the entire procedure was repeated additional times so that each product was assayed on each tooth set. The treatment design was a modified Latin Square design so that no treatment followed another treatment consistently.

The difference between the baseline score and the post-brushing score was calculated for each specimen and represents the polish increment. The mean, standard deviation and standard error of the polish increments was calculated for each group.

Statistical analyses were analyzed using a one-way analysis of variance model [Sigma Plot (11.0) Software]. Since significant differences were found, additional all pair wise comparisons were done using the Student-Newman-Keuls method. All analyses were done with the significance level set at below 0.05. The results are set forth in Table 4 below.

**Table 4**

| Sample ID | High Cleaning Silica (%) | Perlite (%) | Mean Polish Increment (Glossmeter) |
|---|---|---|---|
| Toothpaste sample 1 | 20 | 0 | 24.56 ± 4.23 |
| Toothpaste sample 2 | 18 | 2 | 37.24 ± 3.29 |

Table 4 shows that the natural glass having a smaller top particle size is more effective in tooth polishing compared to a hydrated silica control. The small platy perlite particles are thought to provide effective tooth cleaning and tooth polishing.

Perlite having a smaller top particle size can provide multiple functions in the toothpaste as an abrasive for cleaning and polishing and also as a thickener. Since it is a natural glass, it can be used in the all natural toothpaste formulations.

Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

## Claims

1. A composition for cleaning teeth, the composition comprising natural glass, wherein the natural glass has a top particle size (d₉₀) ranging from 20 *µ*m to less than 50 *µ*m and a median particle size (d₅₀) less than 30 *µ*m, and wherein the natural glass ranges from 0.1 percent to 25 percent by weight of the composition and comprises perlite.

2. The composition of claim 1, wherein the natural glass comprises expanded perlite.

3. The composition of claim 1, wherein the natural glass comprises milled and classified, expanded perlite.

4. The composition of claim 1, wherein the natural glass comprises unexpanded perlite.

5. The composition of claim 1, wherein the natural glass has a top particle size (d₉₀) less than 40 *µ*m, for example less than 35 *µ*m.

6. The composition of claim 1, wherein the natural glass has a top particle size (d₉₀) ranging from 20 *µ*m to 40 *µ*m, for example ranging from 25 *µ*m to 35 *µ*m.

7. The composition of claim 1, wherein the natural glass has a median particle size (dso) less than 25 *µ*m , or ranging from 5 *µ*m to 25 *µ*m.

8. The composition of claim 1, wherein the natural glass has a median particle size (dso) ranging from 10 *µ*m to 20 *µ*m.

9. The composition of claim 1, wherein the natural glass ranges from 0.1 percent to 15 percent by weight of the composition, for example from 0.1 percent to 10 percent by weight of the composition, or from 0.1 percent to 5 percent by weight of the composition.

10. The composition of claim 1, further comprising a toothpaste base, wherein the toothpaste base comprises at least one of binders, humectants, abrasives, detergents, flavoring agents, and preventatives.

## Patentansprüche

1. Zusammensetzung zur Reinigung von Zähnen, wobei die Zusammensetzung mineralisches Glas umfasst, wobei das mineralische Glas eine obere Teilchengröße (d₉₀) von 20 µm bis unter 50 µm und eine mittlere Teilchengröße (d₉₀) von unter 30 µm aufweist und wobei das mineralische Glas zwischen 0,1 Gewichtsprozent bis 25 Gewichtsprozent der Zusammensetzung ausmacht und Perlit umfasst.

2. Zusammensetzung gemäß Anspruch 1, wobei das mineralische Glas Blähperlit umfasst.

3. Zusammensetzung gemäß Anspruch 1, wobei das mineralische Glas gemahlenes und sortiertes Blähperlit umfasst.

4. Zusammensetzung gemäß Anspruch 1, wobei das mineralische Glas unaufgeblähtes Perlit umfasst.

5. Zusammensetzung gemäß Anspruch 1, wobei das mineralische Glas eine obere Teilchengröße (d₉₀) von unter 40 µm aufweist, beispielhaft 35 µm.

6. Zusammensetzung gemäß Anspruch 1, wobei das mineralische Glas eine obere Teilchengröße (d₉₀) von 20 µm bis 40 µm aufweist, beispielhaft 25 µm bis 35 µm.

7. Zusammensetzung gemäß Anspruch 1, wobei das mineralische Glas eine mittlere Teilchengröße (d₅₀) von unter 25 µm oder von 5 µm bis 25 µm aufweist.

8. Zusammensetzung gemäß Anspruch 1, wobei das mineralische Glas eine mittlere Teilchengröße (d₅₀) von 10 µm bis 20 µm aufweist.

9. Zusammensetzung gemäß Anspruch 1, wobei das mineralische Glas zwischen 0,1 Gewichtsprozent und 15 Gewichtsprozent der Zusammensetzung ausmacht, beispielhaft zwischen 0,1 Gewichtsprozent und 10 Gewichtsprozent der Zusammensetzung oder zwischen 0,1 Gewichtsprozent und 5 Gewichtsprozent der Zusammensetzung.

10. Zusammensetzung gemäß Anspruch 1 ferner umfassend: eine Zahnpastabasis, wobei die Zahnpastabasis mindestens Bindemittel, Feuchthaltemittel, Detergenzien, Aromastoffe und/oder Präventivmittel umfasst.

## Revendications

1. Une composition pour le nettoyage des dents, la composition comprenant du verre naturel, dans laquelle le verre naturel a une granulométrie supérieure (d₉₀) qui varie de 20 µm à moins de 50 µm et une granulométrie médiane (d₅₀) inférieure à 30 µm, et dans laquelle le verre naturel varie de 0,1 % à 25 % en poids de la composition et comprend la perlite.

2. La composition selon la revendication 1, dans laquelle le verre naturel comprend de la perlite expansée.

3. La composition selon la revendication 1, dans laquelle le verre naturel comprend de la perlite expansée broyée et classée.

4. La composition selon la revendication 1, dans laquelle le verre naturel comprend de la perlite non expansée.

5. La composition selon la revendication 1, dans laquelle le verre naturel a une granulométrie supérieure (d₉₀) qui est inférieure à 40 µm, par exemple inférieure à 35 µm.

6. La composition selon la revendication 1, dans laquelle le verre naturel a une granulométrie supérieure (d₉₀) qui varie de 20 µm à 40 µm, par exemple qui varie de 25 µm à 35 µm.

7. La composition selon la revendication 1, dans laquelle le verre naturel a une granulométrie médiane (d₅₀) qui est inférieure à 25 µm, ou qui varie de 5 µm à 25 µm.

8. La composition selon la revendication 1, dans laquelle le verre naturel a une granulométrie médiane (d₅₀) qui varie de 10 µm à 20 µm.

9. La composition selon la revendication 1, dans laquelle le verre naturel varie de 0,1 % à 15 % en poids de la composition, par exemple de 0,1 % à 10 % en poids de la composition, ou de 0,1 % à 5 % en poids de la composition.

10. La composition selon la revendication 1, comprenant en outre une base de dentifrice, dans laquelle la base de dentifrice comprend au moins l'un des agents liants, humectants, abrasifs, détergents, aromatisants et préventifs.
